# FASCICULE DE BREVET EUROPEEN

(11) **EP 3 191 106 B1**
(45) Date de publication et mention de la délivrance du brevet: **19.06.2019**
(21) Numéro de dépôt: 15762983.3
(22) Date de dépôt: 11.09.2015
(51) Int. Cl.: A61K 33/00, A61P 3/02, A61K 35/748, A23L 17/60

(54) **COMPOSITION COMPRENANT UNE MICROALGUE ENRICHIE EN SILICIUM POUR UNE UTILISATION THERAPEUTIQUE**
ZUSAMMENSETZUNG MIT SILICIUM-ANGEREICHERTEN MIKROALGEN ZUR THERAPEUTISCHEN VERWENDUNG
COMPOSITION INCLUDING SILICON-ENRICHED MICROALGAE FOR THERAPEUTIC USE

(30) Priorité: 11.09.2014 FR 1458552
(43) Date de publication de la demande: 19.07.2017
(73) Titulaire: Phyco- Biotech, 34070 Montpellier (FR); Université de Montpellier, 34090 Montpellier (FR); Institut National de la Santé et de la Recherche Médicale (INSERM), 75013 Paris (FR)
(72) Inventeur: BACCOU, Jean-Claude, 34080 Montpellier (FR); CRISTOL, Jean-Paul, 34980 Saint Clément de Rivière (FR); GAILLET, Sylvie, 34830 Clapiers (FR); GAY, Gilbert, 34070 Montpellier (FR); JOUY, Nicolas, 34980 St Gely du Fesc (FR); RICHARD, Sylvain, 34270 Saint Mathieu de Treviers (FR); ROUANET, Jean-Max, 34980 Saint Gely du Fesc (FR); VIDE, Joris, 34490 Lignan sur Orb (FR); VIRSOLVY, Anne, 34430 Saint Jean de Vedas (FR)
(74) Mandataire: Argyma
(86) Numéro de dépôt international: PCT/EP2015/070799
(87) Numéro de publication internationale: WO 2016/038175

(56) Documents cités:
- WO-A2-02/15721
- RO-A1- 97 207
- US-A1- 2002 068 110
- Phycobiotech: "Solution articulations | Phycobiotech", , 2013, XP055138443, Extrait de l'Internet: URL:http://www.phyco-biotech.com/fr/soluti on-articulations [extrait le 2014-09-05]
- Phycobiotech: "Solution articulations | Phycobiotech", , 2013, XP055138444, Extrait de l'Internet: URL:http://www.phyco-biotech.com/fr/soluti on-articulations [extrait le 2014-09-05]
- MARTIN KEITH R: "Silicon: the health benefits of a metalloid", METAL IONS IN LIFE SCIENCES, WILEY, UK, vol. 13, 1 janvier 2013 (2013-01-01), pages 451-473, XP009180010, ISSN: 1559-0836, DOI: 10.1007/978-94-007-7500-8_14
- FUSAKO MAEHIRA ET AL: "Soluble silica and coral sand suppress high blood pressure and improve the related aortic gene expressions in spontaneously hypertensive rats", NUTRITION RESEARCH, ELSEVIER INC, XX, vol. 31, no. 2, 7 décembre 2010 (2010-12-07), pages 147-156, XP028165568, ISSN: 0271-5317, DOI: 10.1016/J.NUTRES.2010.12.002 [extrait le 2010-12-13] cité dans la demande
- RISS JEROME ET AL: "Phycobiliprotein C-phycocyanin from Spirulina platensis is powerfully responsible for reducing oxidative stress and NADPH oxidafse expression induced by an atherogenic diet in hamsters", JOURNAL OF AGRICULTURAL AND FOOD CHEMISTRY, AMERICAN CHEMICAL SOCIETY, US, vol. 55, no. 19, 1 septembre 2007 (2007-09-01), pages 7962-7967, XP002587556, ISSN: 0021-8561, DOI: 10.1021/JF070529G [extrait le 2007-08-16] cité dans la demande

## Description

La présente invention a pour objet une composition comprenant une microalgue enrichie en silicium sous une forme soluble dans l'eau destinée à être utilisée pour la prévention ou le traitement d'une pathologie liée au syndrome métabolique. L'invention a également pour objet l'utilisation d'une telle composition pour la prévention ou le traitement d'une pathologie choisie parmi le diabète, l'obésité, les dyslipidémies, les pathologies cardiovasculaires, l'athérosclérose et l'inflammation.

Il est admis aujourd'hui que l'augmentation de la fréquence de surcharge pondérale et de l'obésité, du diabète de type 2 et/ou des maladies cardiovasculaires chez de nombreuses personnes dans les pays développés est due à un apport excessif en calories et en corps gras. Ces pathologies sont liées à une déficience métabolique, ou syndrome métabolique, qui affecte près de 50 millions d'américains (presque un américain adulte sur 4) et environ 30 millions d'européens. Environ 7% des adultes entre 20 et 30 ans et 40% des adultes de plus de 40 ans présentent les critères de ce syndrome métabolique. Le fait de présenter un ou plusieurs des facteurs liés au syndrome métabolique- augmentation de la tension artérielle, taux élevé d'insuline, excès de graisse autour de la taille ou taux anormal de cholestérol - favorise le risque de pathologies graves, notamment l'athérosclérose.

Les compositions actuellement connues utilisées pour traiter ou prévenir ces pathologies et/ou pour limiter un ou plusieurs facteurs de risque sont notamment insuffisamment efficaces et trop chères. Par ailleurs, les régimes hypocaloriques peuvent avoir un succès limité pour l'amélioration de la surcharge pondérale et de l'obésité, et notamment un effet limité dans le temps.

Il existe donc un besoin de nouvelles compositions pour le traitement ou la prévention de pathologies liées au syndrome métabolique.

De manière surprenante, les inventeurs ont mis en évidence que l'administration à des sujets d'une composition comprenant une microalgue enrichie en silicium sous une forme soluble dans l'eau entraîne la réduction, ou le retour à un niveau standard, de certains paramètres modifiés dans le cas de l'absorption d'un régime riche en lipides. Ces résultats ont été obtenus dans un modèle animal reconnu de l'athérosclérose et des dyslipidémies (Vinson *et al*., 2001 ; Wang *et al.,* 2001). En effet, plusieurs paramètres caractérisant les lipides sanguins, le stress oxydatif et l'inflammation, mesurés chez un animal soumis à un régime hypercalorique et recevant une composition selon l'invention, ont un niveau similaire à celui d'un animal témoin soumis à un régime alimentaire standard et ne recevant pas une composition selon l'invention.

Les avantages d'une composition selon l'invention sont :
- La facilité, le faible coût et la reproductibilité de production,
- La stabilité dans le temps,
- La qualité de niveau de qualité alimentaire,
- Un fort effet biologique,
- La possibilité d'une administration par voie orale.

On connaît l'importance du rôle du silicium dans la formation des os en améliorant l'absorption du calcium, la formation du collagène, composant essentiel de l'architecture de la peau et des tissus conjonctifs notamment de la paroi des artères et des cartilages et le bon état des ongles. Le silicium se situe principalement dans le tissu conjonctif, dans les macromolécules comme les glycosaminoglycanes, le collagène et l'élastine (Seabome C.D. *et al.*, 1993). Des taux élevés de silicium liés sont présents dans la paroi artérielle, en particulier dans l'intima. Sur des ostéoblastes en culture *in-vitro*, l'acide orthosilicique stimule la synthèse de collagène de type 1 et la différenciation des ostéoblastes (Reffitt *et al.,* 2003).

La présence de silicium dans le tissu aortique réduit la pression artérielle chez le rat spontanément hypertendu et supprime l'expression des gènes de l'angiogénèse dans la paroi aortique et des facteurs de croissance liés au remodelage vasculaire (Maehira *et al.,* 2011). Un effet anti-athéromateux de silicium administré par voie intraveineuse ou *per os* a été montré chez le lapin (Loeper *et al.,* 1979). Dans cette publication, les auteurs utilisent du silicate de sodium, du silicate de lysine et du monométhyltrisilanol administrés quotidiennement durant l'étude soit par voie intraveineuse soit *per os.* Ces formes de silicium ne correspondent pas à celles présentes dans la composition comprenant une microalgue enrichie en silicium. De plus il est inenvisageable d'administrer ces formes de silicium en intraveineux chez l'homme. Or c'est sous cette forme d'administration que des résultats positifs ont été observés sur le nombre de plaques d'athéromes présentes sur les aortes des lapins traités. Cependant, même sur ces lapins recevant ces formes de silicium par voie intraveineuse, les auteurs observent une variation non significative des lipides plasmatiques des animaux ayant reçu du silicium. L'utilisation d'une composition comprenant une microalgue enrichie en silicium pour améliorer les caractéristiques de lipides sanguins, le stress oxydatif et/ou l'inflammation créés par l'absorption d'un régime hypercalorique ne sont pas décrits.

L'invention sera maintenant décrite de manière détaillée et à l'aide d'exemples, destinés à illustrer l'invention sans aucunement en limiter la portée.

La présente invention a pour premier objet une composition comprenant une microalgue enrichie en silicium sous une forme soluble dans l'eau, destinée à être utilisée selon la revendication 1.

Le terme « microalgue » désigne une algue microscopique photosynthétique, ladite microalgue peut être une microalgue eucaryote ou procaryote.

Le terme «microalgue enrichie en silicium » désigne une microalgue associée à du silicium présent en quantité supérieure par rapport à la quantité de silicium habituellement mesurée en association avec ladite microalgue lorsqu'elle est cultivée dans des conditions standard ou qu'elle est isolée de son milieu naturel.

Une composition selon l'invention comprend une microalgue enrichie en silicium dans laquelle le silicium peut être détecté dans le compartiment intracellulaire et/ou associé aux membranes et/ou associé à la surface de ladite microalgue. Le silicium peut être détecté par tout moyen connu de l'homme du métier, notamment par l'observation microscopique, et de préférence par microscopie électronique couplée à une microsonde à rayons X. Lors de l'observation par microscopie électronique couplée à une microsonde à rayons X, des grains de silicium particulièrement denses peuvent être observés.

Le terme « silicium sous une forme soluble dans l'eau », ou éventuellement « silicium soluble dans l'eau », désigne du silicium présent et détectable dans un extrait aqueux d'une composition selon l'invention. Selon un aspect particulier de l'invention, ledit silicium sous une forme soluble dans l'eau est présent dans un extrait aqueux d'une composition selon l'invention comprenant une microalgue enrichie en silicium. La détection de la présence de silicium associé à une microalgue dans une composition selon l'invention peut être réalisée également par tout moyen chimique ou biochimique connu de l'homme du métier.

Le terme «silicium sous une forme soluble dans l'eau» ou « silicium sous une forme soluble » ou « silicium solubilisable » désigne du silicium disponible sous une forme réduite et pouvant être assimilé par l'organisme, contrairement au silicium minéral, qui est sous une forme oxydée, telle que la silice, qui peut précipiter et n'est pas assimilable par l'organisme. Dans une composition selon l'invention, le silicium présent sous une forme soluble dans l'eau peut se présenter sous une forme simple ou sous une forme complexée à une molécule organique.

Le terme « pathologie liée au syndrome métabolique » désigne l'une des pathologies pouvant être observée et participer au syndrome métabolique. Le syndrome métabolique représente un ensemble de facteurs qui augmentent le risque de maladies cardio-vasculaires, d'attaques cérébrales et de diabète de type 2. Le terme « syndrome métabolique » peut désigner aussi le syndrome cardiométabolique ou le prédiabète. La prévention d'une pathologie liée au syndrome métabolique désigne également l'amélioration du bien-être d'un consommateur dont l'alimentation est riche en calories et/ou en corps gras, et qui de ce fait présente un risque de stress oxydant et/ou de maladie cardiovasculaire.

Dans un rapport de 2004 intitulé « Global health risks: mortality and burden of disease attributable to selected major risks », l'Organisation Mondiale de la Santé (OMS), a évalué l'impact de 24 facteurs de risques sur la mortalité à l'échelle mondiale, en compilant des données scientifiques et sanitaires provenant de tous les pays, industrialisés ou non. Parmi les facteurs de risque principaux, l'OMS indique que les facteurs « hypertension », « un taux de glycémie élevée », « inactivité physique », « surpoids et obésité » et un taux élevé de cholestérol » totalisent 33,4% des décès au niveau planétaire, tous pays confondus. Or ces facteurs de risque correspondent au syndrome métabolique.

Selon un aspect particulier, une composition selon l'invention comprend une microalgue enrichie en silicium sous une forme soluble dans l'eau, ladite microalgue étant choisie parmi les microalgues eucaryotes. Selon un aspect plus particulier de l'invention, ladite microalgue eucaryote est choisie dans le groupe constitué par : les Chlorophycées (et de préférence *Dunaliella*, *Chlorella ou Parietochloris incisa),* les Chrysophycées, les Coccolithophyceae, les Diatomées, les Euglénophycées, les Rhodophycées et les Trebouxiophyceae.

Selon un autre aspect particulier, une composition selon l'invention comprend une microalgue enrichie en silicium sous une forme soluble dans l'eau, ladite microalgue étant choisie parmi les microalgues procaryotes, aussi désignées par le terme « cyanobactéries » ou « algues bleues ». Selon un aspect plus particulier de l'invention, ladite microalgue procaryote est choisie dans le groupe constitué par les cyanophycées, parmi lesquelles la spiruline est particulièrement préférée.

Selon un aspect particulier, l'invention a donc pour objet une composition comprenant une microalgue enrichie en silicium sous une forme soluble dans l'eau, ladite microalgue étant la spiruline, ladite composition étant destinée à être utilisée pour la prévention ou le traitement d'une pathologie liée au syndrome métabolique, telle que définie ci-dessus.

Le terme « spiruline » désigne une algue filamenteuse bleue-verte appartenant au phyllum Cyanophyta, de la classe des Cyanophyceae, de l'ordre Nostocales, famille Oscillatoriaceae, et du genre Spirulina ou Arthrospira, et de préférence *Arthrospira platensis* ou *Arthrospira maxima.*

Les spirulines sont des microalgues très riches en protéines, de l'ordre de 70% de protéines par rapport à la matière sèche, qui sont un complément nutritionnel reconnu pour son efficacité et autorisées en alimentation humaine. Les spirulines sont souvent cultivées dans des bassins, ou éventuellement dans des réacteurs tubulaires, et ceci permet de contrôler les conditions de culture donc d'obtenir des résultats reproductibles quant à la composition de la matière végétale obtenue, par comparaison avec des cultures de végétaux réalisées en plein champ ou même sous serre.

Selon un aspect plus particulier, la présente invention a pour objet une composition comprenant une microalgue enrichie en silicium sous une forme soluble dans l'eau destinée à être utilisée pour la prévention ou le traitement d'une pathologie liée au syndrome métabolique, ladite microalgue étant choisie parmi *Arthrospira platensis* et *Arthrospira maxima.*

Selon un aspect particulier, l'invention a pour objet une composition comprenant une microalgue enrichie en silicium sous une forme soluble dans l'eau, destinée à être utilisée comme médicament.

Selon un autre aspect particulier, l'invention a pour objet une composition comprenant une microalgue enrichie en silicium sous une forme soluble dans l'eau, destinée à être utilisée comme composition pharmaceutique, comme nutraceutique ou comme complément alimentaire, pour la prévention ou le traitement d'une pathologie liée au syndrome métabolique telle que définie précédemment.

Une composition selon l'invention peut comprendre une composition comprenant une microalgue enrichie en silicium sous une forme soluble dans l'eau, et optionnellement tout véhicule ou excipient approprié, acceptable du point de vue pharmaceutique, cosmétique, alimentaire ou nutraceutique, ainsi que des additifs conventionnels, connus de l'homme du métier.

Selon un aspect particulier, une composition selon l'invention est formulée pour être administrée par voie orale. Selon un autre aspect particulier, une composition selon l'invention se présente sous la forme de gélule, capsule, comprimé, granule, tablette, poudre ou solution buvable. Une composition selon l'invention formulée pour être administrée par voie orale en tant que complément alimentaire peut se présenter sous forme d'une composition comprenant une microalgue enrichie en silicium sous une forme soluble dans l'eau, selon l'invention, ou bien sous forme de gélules ou de capsules molles de gélatine ou végétales. Ledit complément alimentaire peut alors contenir de 10 à 100% en poids de d'une composition comprenant une microalgue enrichie en silicium sous une forme soluble dans l'eau.

Les modes d'administration, les posologies et les formes galéniques optimales des composés et compositions selon l'invention peuvent être déterminés selon les critères généralement pris en compte dans l'établissement d'un traitement pharmaceutique, en particulier dermatologique, cosmétique ou vétérinaire adapté à un patient ou à un animal comme par exemple l'âge ou le poids corporel du patient ou de l'animal, la gravité de son état général, la tolérance au traitement, les effets secondaires constatés, le type de peau. En fonction du type d'administration souhaitée, la composition et/ou les composés actifs selon l'invention peuvent en outre comprendre au moins un excipient pharmaceutiquement acceptable, ou un excipient cosmétiquement acceptable.

La composition selon la présente invention peut en outre comprendre au moins un adjuvant pharmaceutique ou cosmétique connu de l'homme du métier, choisi parmi les épaississants, les conservateurs, les parfums, les colorants, des filtres chimiques ou minéraux, les agents hydratants, les eaux thermales, etc.

L'invention a pour objet une composition comprenant une microalgue enrichie en silicium sous une forme soluble dans l'eau, destinée à être utilisée comme médicament pour son utilisation pour la prévention ou le traitement d'une pathologie liée au syndrome métabolique, ladite pathologie étant choisie parmi :
- Le diabète de type 2,
- L'obésité nutritionnelle,
- Les dyslipidémies,
- Les pathologies cardiovasculaires,
- L'athérosclérose, et
- L'inflammation.

Le diabète de type 2 se caractérise par une hyperglycémie chronique. Selon un aspect particulier, une composition selon l'invention est utilisée comme médicament pour prévenir ou traiter une dysrégulation de la sécrétion de l'insuline et/ou pour la prévention ou le traitement de l'insulino-résistance.

L'obésité correspond à un excès de masse grasse.

Le terme « dyslipidémie » désigne un taux anormal de lipides dans le sang. Ce terme inclut notamment les hyperlipidémies, c'est à dire une élévation du taux de lipides sanguins observée lors d'une alimentation riche en graisses. Parmi les hyperlipidémies, on peut citer :
- Une élévation du taux de lipoprotéines choisies parmi les chylomicrons, les IDL (intermediate density lipoprotein), les HDL (high density lipoproteins), les LDL (low density lipoproteins).
- Une élévation du taux de cholestérol total,
- Une élévation du taux de triglycérides et
- Une combinaison de deux ou plus de ces paramètres.

Selon un autre aspect particulier, l'invention a pour objet une composition comprenant une microalgue enrichie en silicium sous une forme soluble dans l'eau, destinée à être utilisée comme médicament pour la prévention ou le traitement d'une pathologie cardiovasculaire. Le terme « pathologie cardiovasculaire » désigne une pathologie affectant le système le coeur et la circulation sanguine d'un sujet, et notamment les maladies coronariennes, les maladies du muscle cardiaque, des valves cardiaques ou du péricarde, les maladies du rythme ou de la conduction cardiaque, les maladies des vaisseaux, les pathologies artérielles (hypertension et hypotension artérielle), les troubles de la fonction contractile artérielle, l'athérome et l'athérosclérose. En effet, les inventeurs ont montré que l'administration à un sujet d'une composition selon l'invention permet d'observer un effet vasculoprotecteur.

Selon un autre aspect particulier, l'invention a pour objet une composition comprenant une microalgue enrichie en silicium sous une forme soluble dans l'eau, destinée à être utilisée pour la prévention ou le traitement d'une inflammation, et/ou d'une inflammation de bas niveau.

Selon un autre aspect particulier, l'invention a pour objet une composition comprenant une microalgue enrichie en silicium sous une forme soluble dans l'eau, destinée à être utilisée pour la prévention ou la diminution de la surcharge pondérale, pour la prévention ou la diminution de l'adipogenèse, ou en tant qu'agent anti-oxydant.

Selon un autre aspect particulier, l'invention a pour objet une composition comprenant une microalgue enrichie en silicium sous une forme soluble dans l'eau, destinée à être utilisée comme médicament pour la prévention ou le traitement d'une pathologie associée à, ou consécutive à, un stress oxydant. Le « stress oxydant » est notamment caractérisé par un état comprenant la surproduction d'espèces réactives de l'oxygène, par exemple les anions superoxyde.

Selon un autre aspect, une composition selon l'invention comprend une microalgue enrichie en silicium sous une forme soluble dans l'eau, destinée à être utilisée pour la prévention ou le traitement d'une pathologie liée au syndrome métabolique, dans laquelle la proportion de silicium total dans ladite composition, par rapport à la masse sèche de microalgue présente dans ladite composition, est comprise entre 0,05% et 1, 2, 3, 4, 5, 6, 7, 8, 9 ou 10%, et de préférence entre 0,1% et 5%, de préférence entre 0,5% et 5%, de préférence entre 0,9% et 4%, entre 0,9% et 3%, entre 0,9% et 2% et plus préférentiellement entre 0,9% et 1,5%.

Le terme « silicium total» désigne la quantité totale du silicium détectée dans une composition selon l'invention. Selon un aspect particulier de l'invention, le terme « silicium total» désigne la quantité totale du silicium détectée dans la masse sèche de microalgue comprise dans une composition destinée à être utilisée pour la prévention ou le traitement d'une pathologie liée au syndrome métabolique, selon l'invention. La quantité totale de silicium peut être déterminée par toute méthode connue de l'homme de l'art après minéralisation totale de l'échantillon de composition selon l'invention, notamment par dosage gravimétrique, par ICP-OES (Inductively Coupled Plasma-Optical Emission Spectrometry) ou par ICP-MS (Inductively Coupled Plasma-Mass Spectrum).

Le terme «masse sèche de microalgue » désigne la masse de microalgue présente dans la composition, déterminée après déshydratation de la microalgue, la déshydratation, ou le séchage, de ladite microalgue pouvant être réalisée par tout protocole choisi parmi les protocoles connus par l'homme de l'art. Le terme de « masse sèche de microalgue » peut être considéré comme équivalent au terme biomasse.

Selon un aspect particulier, une composition selon l'invention comprend une microalgue enrichie en silicium sous une forme soluble dans l'eau, destinée à être utilisée pour la prévention ou le traitement d'une pathologie liée au syndrome métabolique, dans laquelle la proportion de silicium sous une forme soluble dans l'eau dans ladite composition, par rapport à quantité de silicium total présent dans la composition, est comprise entre 5% et 90%, de préférence entre 10% et 80%, de préférence entre 15% et 75%, de préférence entre 20% et 70%, de préférence entre 25% et 65%, de préférence entre 30% et 60%, de préférence entre 35% et 65%, de préférence entre 40% et 60% du silicium total présent dans ladite composition.

Dans ladite composition selon l'invention, la proportion de silicium sous une forme soluble dans l'eau peut être déterminée par tout moyen connu de l'homme du métier, et notamment par l'extraction de ladite composition selon l'invention par une solution aqueuse. De préférence, la proportion de silicium sous une forme soluble dans l'eau dans une composition selon l'invention peut être déterminée par extraction séquentielle de la composition par des solutions aqueuses d'agents actifs d'une composition selon l'invention. Si le silicium est présent sous une forme soluble dans l'eau, il sera solubilisé partiellement ou totalement au cours des extractions successives. Si le silicium est présent sous une forme polymérisée, il restera sous une forme insoluble malgré les extractions successives. L'extraction peut notamment être réalisée par : de l'eau, une solution enzymatique capable de dégrader les parois cellulaires végétales, une solution de détergent capable de solubiliser les fractions lipophiles, ou une solution enzymatique capable de digérer les protéines. La quantité de silicium présente dans un extrait aqueux, ou dans tout extrait d'une composition destinée à être utilisée pour la prévention ou le traitement d'une pathologie liée au syndrome métabolique, selon l'invention, peut être déterminée par toute méthode connue de l'homme de l'art, notamment par dosage gravimétrique, par ICP-OES (Inductively Coupled Plasma-Optical Emission Spectrometry) ou par ICP-MS (Inductively Coupled Plasma-Mass Spectrum).

Selon un autre aspect, l'invention a pour objet une composition comprenant une microalgue enrichie en silicium sous une forme soluble dans l'eau, destinée à être utilisée pour la prévention ou le traitement d'une pathologie liée au syndrome métabolique telle que définie ci-dessus, ladite composition étant obtenue par la mise en oeuvre d'un procédé comprenant les étapes suivantes :
a) la mise en culture de ladite microalgue dans des conditions et un milieu de culture adaptés jusqu'à l'obtention d'une culture de microalgue caractérisée par une biomasse sèche comprise entre 0,1 et 2 g/litre de culture,
b) la préparation d'une solution aqueuse de silicium, par l'ajout de silicium sous la forme d'une solution ou d'une poudre, suivie de l'agitation de la solution aqueuse jusqu'à dissolution complète du silicium,
c) l'addition de la solution préparée en b) à la culture de microalgue obtenue en a),
d) la mise en culture de ladite microalgue sous agitation pendant une durée d'au moins 12 heures,
e) la récolte d'au moins une partie de la culture de microalgue.

Selon un aspect plus particulier, l'invention a pour objet une composition comprenant une microalgue enrichie en silicium sous une forme soluble dans l'eau, destinée à être utilisée pour la prévention ou le traitement d'une pathologie liée au syndrome métabolique telle que définie ci-dessus, ladite composition étant obtenue par la mise en oeuvre d'un procédé comprenant la mise en culture de ladite microalgue dans des conditions et un milieu de culture adaptés jusqu'à l'obtention d'une culture de microalgue caractérisée par une biomasse sèche comprise entre 0,1 et 2 g/litre de culture, suivie de la préparation d'une solution aqueuse de silicium et de polyphénols, par l'addition de polyphénols à une solution aqueuse de silicium ou par l'addition de silicium à une solution aqueuse de polyphénols, suivie d'un mélange par agitation.

Dans un procédé de préparation d'une composition comprenant une microalgue enrichie en silicium sous une forme soluble dans l'eau selon l'invention, la culture de microalgues est réalisée dans des conditions adaptées à la culture des microalgues et connues de l'homme du métier. La culture des microalgues est réalisée dans des conditions permettant d'éviter les contaminations atmosphériques, notamment sous serre et dans un bassin permettant une pénétration optimale de la lumière, et donc l'activité photosynthétique, par exemple dans des bassins de 25 M³ et d'une faible profondeur, entre 15 et 25 cm, et de préférence 20 +/- 3 cm. En effet, le risque de récolter la spiruline croissant naturellement dans des lacs est de voir proliférer certaines cyanobactéries toxiques dans ces conditions non contrôlées.

Le mode de culture le plus répandu et le moins coûteux pour la spiruline est le bassin de type « raceway » dont l'agitation est assurée par une roue à aubes et avec une cloison centrale permettant de faire circuler la culture en circuit fermé. Ce mode de production permet d'isoler la culture dans des bassins de faible profondeur où les apports minéraux du milieu de culture artificiel sont contrôlés.

La culture est possible en cuve de 1 M³ avec le même milieu de culture et agitation par bullage à l'air comprimé ou par pompe. Un autre mode de culture est possible dans un photo réacteur tubulaire, essentiellement utilisé pour la culture de microalgues unicellulaires comme la chlorelle.

La culture de microalgues est généralement réalisée à un pH compris entre pH6 et pH12, de préférence à pH alcalin, plus préférentiellement à un pH compris entre pH9 et pH11, et plus préférentiellement à pH10. La culture peut être réalisée sous serre, en éclairage naturel et/ou artificiel, permettant de réaliser la photosynthèse, dans des conditions contrôlées de température, de pH et d'alimentation en dioxyde de carbone. L'agitation est réalisée par une ou des pompes, une roue à aubes ou un bullage par de l'air comprimé pour assurer la circulation de la culture et un brassage en profondeur. La température de culture est comprise entre 15 et 40°C avec un optimum à 33°C.

La durée de l'étape de culture de la microalgue, dans des conditions et un milieu de culture adapté, jusqu'à obtention d'une culture caractérisée par une biomasse sèche comprise de préférence entre 0,4 et 0,6 g/litre, dépend de la concentration de l'ensemencement initial et des conditions de lumière et de température appliquées à la culture, elle est typiquement comprise entre 3 et 21 jours.

Dans l'étape de préparation d'une solution aqueuse de silicium, la dissolution de silicium est réalisée dans une solution aqueuse ou dans de l'eau, et de préférence de l'eau dé-ionisée ou purifiée c'est-à-dire ne contenant pas ou très peu de minéraux, et dans un volume restreint par rapport au volume final de la culture de microalgue, le volume d'eau dans lequel est dissous le silicium est typiquement compris entre 1/20^{ème} et 1/30^{ème} du volume final de culture. L'eau peut être filtrée et stérilisée par irradiation UV avant d'être utilisée pour la préparation de la solution et versée dans le bassin.

Le silicium sous forme soluble se trouve essentiellement sous forme d'acide orthosilicique Si(OH)₄ dans les eaux de surface. A une concentration supérieure à 2mM et à pH neutre, l'acide orthosilicique polymérise en différentes formes de silice, de colloïdale à solide peu ou pas soluble. Il est donc essentiel de le maintenir en solution afin qu'il reste absorbable par la microalgue, bien que le pH préférentiellement alcalin du milieu de culture d'une microalgue soit favorable à la solubilisation de l'acide orthosilicique.

Le silicium peut être ajouté à la solution aqueuse sous la forme d'une solution ou d'une poudre, avec agitation jusqu'à dissolution complète du silicium.

Le silicium peut aussi être ajouté sous une forme combinée, par exemple sous la forme de silicate. En particulier, le silicium peut être ajouté sous la forme d'une composition choisie parmi :
- du métasilicate de sodium ou du métasilicate de potassium, qui se dissout totalement dans l'eau et libère de l'acide orthosilicique en solution. Le métasilicate de sodium ou potassium se présente sous forme de poudre ou de solution aqueuse concentrée.
- un engrais ou toute composition contenant du silicium dont tout ou partie est sous forme soluble d'acide orthosilicique.
- un amendement silicique utilisé en agriculture.

Dans le cas d'une composition sous forme de poudre, celle-ci sera ajoutée sous la forme d'une poudre dont la taille des particules est inférieure à 30 microns, de préférence inférieure à 20 microns et plus préférentiellement inférieure à 10 microns. Ceci permet, lors de la récolte des spirulines par tamisage sur une toile filtrante de maille 30 µm, que les particules soient éliminées lors du tamisage, limitant ainsi la présence de silicium sous forme minérale dans la composition comprenant les spirulines. Le silicium peut être ajouté sous la forme d'un engrais de type Agrosil broyé, caractérisé par des particules de taille inférieure à 10 microns.

Une composition comprenant une microalgue enrichie en silicium sous une forme soluble dans l'eau, destinée à être utilisée pour la prévention ou le traitement d'une pathologie liée au syndrome métabolique, selon l'invention, peut être obtenue par la mise en oeuvre d'un procédé comprenant les étapes suivantes :
a) la mise en culture de microalgues dans des conditions et un milieu de culture adaptés, jusqu'à l'obtention d'une culture caractérisée par une biomasse sèche comprise entre 0,1 et 2 g/litre, de préférence entre 0,2 et 1 g/litre, de préférence entre 0,3 et 0,8 g/litre, et de préférence entre 0,4 et 0,6 g/litre de culture,
b) la préparation d'une solution aqueuse de silicium et de polyphénols, le silicium étant ajouté sous la forme d'une solution, ou d'une poudre, et les polyphénols étant ajoutés sous la forme d'une solution, suivie d'un mélange par agitation de la solution ainsi obtenue jusqu'à dissolution complète du silicium et des polyphénols,
c) l'addition de la solution préparée en b) à la culture de microalgues obtenue en a),
d) la mise en culture des microalgues sous agitation pendant une durée d'au moins 12 heures,
e) la récolte d'au moins une partie de la culture de microalgues.

La préparation d'une solution aqueuse de silicium et de polyphénols, à l'étape b), peut être réalisée par l'addition de silicium à la solution aqueuse, suivie de l'addition de polyphénols, ou bien par l'addition de polyphénols à la solution aqueuse, suivie de l'addition de silicium.

Le terme « polyphénols » désigne un composé comprenant un ou plusieurs cycles benzéniques auxquels sont liés un ou plusieurs groupes hydroxyle, les groupes hydroxyle pouvant être libres ou engagés dans une autre fonction, ce terme désigne donc un grand nombre de composés possibles, regroupant des molécules simples telles que les acides phénoliques à des composés hautement polymérisés comme les tanins, et sont caractérisés par un pouvoir anti-oxydant élevé.

Les polyphénols peuvent notamment être choisis parmi : les phénols simples tels que l'hydroquinone, les acides hydroxybenzoïques, les acides hydroxycinnamiques, les coumarines, les naphtoquinones, les stilbénoïdes, les flavonoïdes, les isoflavonoïdes, les anthocyanes, les lignanes, les lignines, les tanins condensés.

Les polyphénols sont présents dans diverses substances naturelles. C'est le cas notamment de l'anthocyanine dans les fruits rouges, des proanthocyanidines dans le chocolat et le vin, d'acides cafeoylquinique et feruloylquinique dans le café, de flavonoïdes dans les agrumes, de catéchines dans le thé vert et de quercétine dans les pommes.

Les composés phénoliques ont la propriété de se complexer partiellement avec les fonctions hydroxyles ionisées de l'acide orthosilicique en solution et ainsi de ralentir la polymérisation de l'acide orthosilicique.

L'addition de la solution de silicium et de polyphénols à la culture de microalgues conduit à une culture de microalgues dans laquelle la concentration en silicium est comprise entre 0,1 et 20 g/litre de culture, entre 0,5 et 4 g/litre de culture, entre 1 et 3 g/litre de culture et de préférence de 2 g/litre de culture, et la concentration en polyphénols est comprise entre 1 et 300 mg/litre de culture, de préférence entre 5 et 60 mg/litre de culture.

La solution de polyphénols ajoutée peut être un extrait de polyphénols naturels. En particulier, un tel extrait de polyphénols naturels peut être choisi parmi des extraits végétaux, et notamment choisi parmi :
- un extrait de polyphénols d'olives, notamment un extrait de margines d'olive,
- un extrait de polyphénols de feuilles de thé,
- un extrait de polyphénol de raisins, notamment un extrait de marc et/ou de pépins de raisin, le raisin pouvant être blanc ou rouge,
- un extrait de polyphénol de gingembre ou de romarin, et
- un mélange de ces extraits.

Ces produits se présentent sous forme de poudre de solubilité variable dans l'eau ou sous forme aqueuse concentrée. Les polyphénols d'olive ou de raisin présentent l'avantage d'être produits localement en grandes quantités et constituent des sous-produits peu coûteux des filières viticoles et oléicoles.

Selon un autre aspect particulier d'un procédé de préparation d'une composition comprenant une microalgue enrichie en silicium sous une forme soluble dans l'eau selon l'invention, la solution de polyphénols comprend au moins un polyphénol d'origine synthétique. Une préparation d'acide orthosilicique stabilisée par de la choline a été autorisée comme complément alimentaire par l'EFSA (The EFSA Journal, 2009, 948, 1-23). La choline pourrait donc être utilisée comme composé permettant de maintenir l'acide orthosilicique en solution. Cependant, la choline est un produit assez coûteux et il faudrait en utiliser des quantités importantes par bassin de 25M³ en substitution des polyphénols de raisin, d'olive, de thé ou d'autres plantes.

Selon un aspect particulier d'un procédé de préparation d'une composition comprenant une microalgue enrichie en silicium sous une forme soluble dans l'eau selon l'invention, les microalgues sont récoltées par tamisage, centrifugation ou floculation, et de préférence par tamisage. Selon un aspect préféré, la microalgue est récoltée par tamisage sur une toile filtrante de maille comprise entre 20 et 40 microns, et de préférence de 30 microns.

Dans un procédé de préparation d'une composition comprenant une microalgue enrichie en silicium sous une forme soluble dans l'eau selon l'invention, la récolte d'une fraction de la culture de microalgue commence après un temps d'incubation de la microalgue avec le silicium, et éventuellement les polyphénols compris entre 12H et 3 jours ; durant cette période, la biomasse de spiruline augmente d'au moins 20%. Selon un aspect particulier d'un procédé selon l'invention, la récolte de fractions successives de la culture de microalgue est ensuite réalisée tous les jours, selon l'état de la culture et ceci pendant une durée de 2 à 8 semaines, préférentiellement environ 3 semaines. Selon un autre aspect particulier d'un procédé selon l'invention, une nouvelle solution aqueuse de silicium, comprenant préférentiellement des polyphénols, est ajoutée environ 3 semaines après la récolte, dans le bassin de culture, pour un nouveau cycle d'enrichissement.

La quantité de microalgue récoltée chaque jour est déterminée pour ne pas mettre en péril toute la culture. Cette quantité varie de 5% à 15% de la quantité totale de microalgue dans la culture. Après chaque récolte partielle, une quantité calculée d'éléments minéraux du milieu de Zarouk, ou de tout autre milieu de culture utilisable pour ce procédé, est ajoutée dans le bassin afin de compenser les éléments minéraux consommés par les microalgues récoltées. Il est aussi possible de récolter la totalité des microalgues du bassin au bout de 8 jours de culture.

Selon un aspect particulier d'un procédé de préparation d'une composition comprenant une microalgue enrichie en silicium sous une forme soluble dans l'eau selon l'invention, la microalgue récoltée est filtrée puis séchée pour obtenir une composition de microalgue déshydratée ou partiellement déshydratée. De préférence, le séchage est effectué à une température inférieure à 60°C, pour éviter de dénaturer les principes actifs contenus dans les compositions de microalgue. Selon un aspect encore plus particulier d'un procédé selon l'invention, la poudre séchée de microalgue est broyée.

Selon un aspect particulier d'un procédé de préparation d'une composition comprenant une microalgue enrichie en silicium sous une forme soluble dans l'eau selon l'invention, après l'addition de la solution aqueuse de silicium, la concentration de silicium dans le milieu de culture lors de l'étape c) est comprise entre 0,1 et 20 g/litre de culture, entre 0,5 et 4 g/litre, entre 1 et 3 g/litre, et de préférence de 2 g/litre. L'apport des 2g/litre de silicium peut être réalisé en une seule fois ou de façon séquentielle, par exemple en 2 jours consécutifs en apportant 1g/litre de silicium le premier jour et 1g/litre de silicium le deuxième jour.

La concentration en polyphénols dans la culture est comprise entre 5 et 60 mg/litre, de préférence entre 10 et 20 mg/litre et plus préférentiellement de 12 mg/litre de culture.

L'apport des 2g/litre de silicium et de polyphénols peut être réalisé en une seule fois ou de façon séquentielle, par exemple en 2 jours consécutifs en apportant 1g/litre de silicium et de polyphénols le premier jour et 1g/litre de silicium et de polyphénols le deuxième jour.

Selon un autre aspect particulier l'invention a pour objet une composition comprenant une microalgue enrichie en silicium sous une forme soluble dans l'eau destinée à être utilisée pour la prévention ou le traitement d'une pathologie liée au syndrome métabolique telle que définie précédemment, caractérisée en ce que la dose de silicium administrée à un sujet est comprise entre 0,3 et 1 mg/kg de poids corporel/jour, de préférence entre 0,4 et 0,8 mg/kg de poids corporel/jour, et plus préférentiellement entre 0,5 et 0,7 mg/kg de poids corporel/jour. La dose de silicium ou quantité de silicium, administrée est la quantité de silicium total, calculée par gravimétrie.

Un autre objet de l'invention consiste en l'utilisation d'une composition comprenant une microalgue enrichie en silicium sous une forme soluble dans l'eau pour la prévention ou le traitement d'une pathologie choisie parmi: le diabète de type 2, l'obésité nutritionnelle, les dyslipidémies, les pathologies vasculaires et l'inflammation.

Plus particulièrement, l'invention a pour objet l'utilisation d'une composition comprenant une microalgue enrichie en silicium sous une forme soluble dans l'eau pour la prévention ou le traitement d'une pathologie choisie parmi: le diabète de type 2, l'obésité nutritionnelle, les dyslipidémies, les pathologies vasculaires et l'inflammation, dans laquelle le patient traité présente un syndrome métabolique ou un diabète de type 2.

Un autre objet de l'invention consiste en l'utilisation d'une composition comprenant une microalgue enrichie en silicium sous une forme soluble dans l'eau pour la prévention ou le traitement de la surcharge pondérale, pour la prévention ou la diminution de l'adipogenèse ou en tant qu'agent anti-oxydant, pour la régulation de la sécrétion d'un agent choisi parmi : le TNF-α, l'IL-6, NFκB, MCP-1 plasmatique, pour la prévention ou le traitement de l'inflammation de bas niveau, ou pour la prévention ou le traitement d'une pathologie liée au stress oxydatif.

Enfin, selon un aspect encore plus particulier, l'invention a pour objet l'utilisation d'une composition comprenant une microalgue enrichie en silicium sous une forme soluble dans l'eau pour la prévention ou le traitement d'une pathologie liée au syndrome métabolique, dans laquelle ladite microalgue est la spiruline.

La présente invention sera maintenant décrite dans des exemples, destinés à illustrer l'invention sans en diminuer la portée.

### LEGENDES DES FIGURES

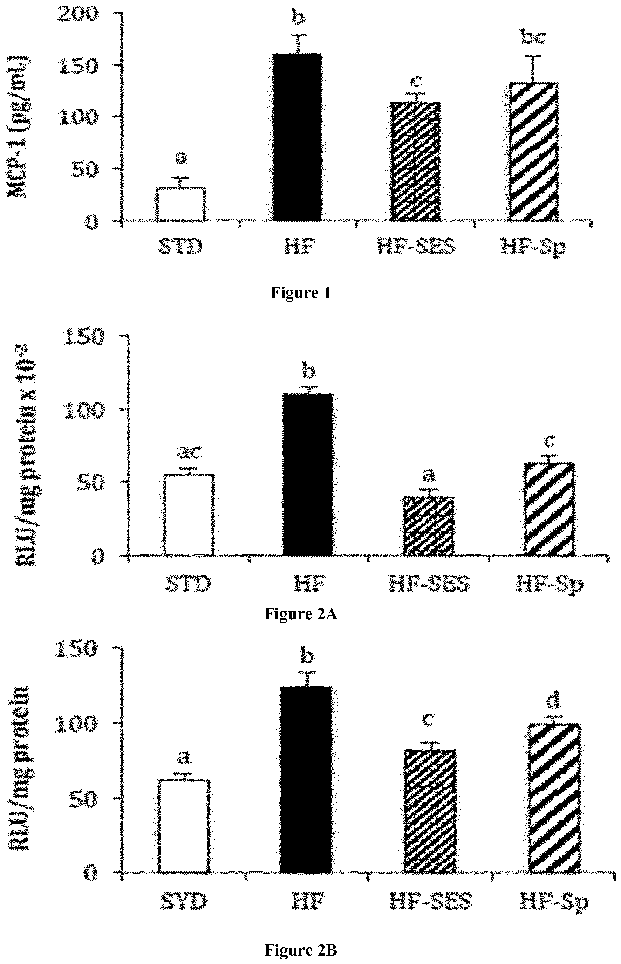

**Figure 1** : Histogramme représentant le niveau de MCP-1 (monocyte chemoattractant protein-1, en pg/mL) dans le plasma de hamsters nourris avec un régime alimentaire standard (STD), un régime enrichi en graisses (HF pour High-Fat), un régime HF additionné de spiruline enrichie en silicium (HF-SES pour Silicon Enriched Spirulina) ou un régime HF additionné de spiruline non enrichie en silicium (HF-Sp pour Spirulina), pendant 12 semaines. Les valeurs indiquent la moyenne +/- SEM (n=6). Pour chacun des régimes diététiques, les barres avec différentes lettres en index sont significativement différentes (p<0,05).
**Figures 2A et 2B** **:** Niveau de NADPH oxydase hépatique (Figure 2A) ou cardiaque (Figure 2B) dans le plasma de hamsters ayant reçu un régime alimentaire standard STD, HF, HF-SES ou HF-Sp pendant 12 semaines. Les valeurs indiquent la moyenne +/- SEM (n=6). Pour chacun des régimes diététiques, les barres avec différentes lettres en index sont significativement différentes (p<0,05).

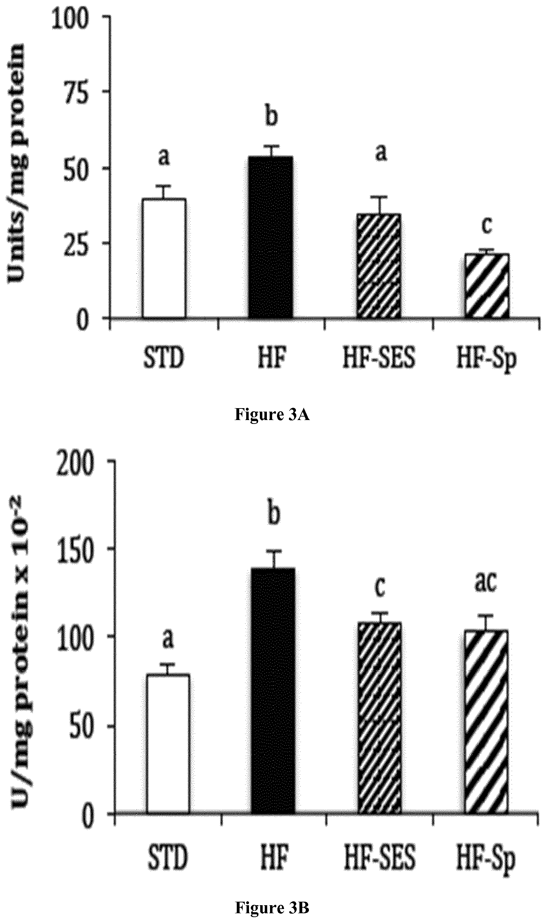

**Figures 3A et 3B** **:** Niveau de superoxyde dismutase (Figure 3A) ou glutathion peroxydase (Figure 3B) dans le plasma de hamsters ayant reçu un régime alimentaire STD, HF, HF-SES ou HF-Sp pendant 12 semaines. Les valeurs indiquent la moyenne +/- SEM (n=6). Pour chacun des régimes diététiques, les barres avec différentes lettres en index sont significativement différentes (p<0,05).

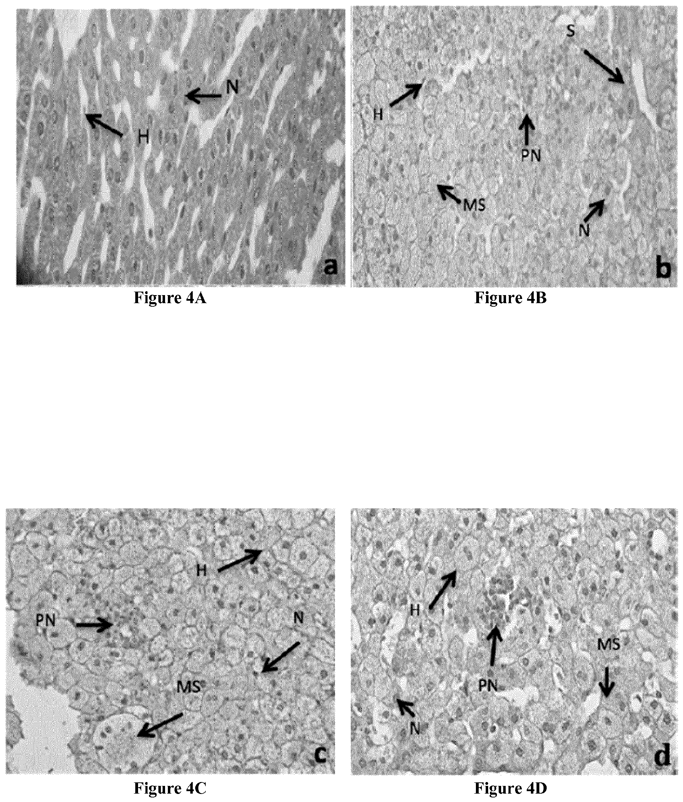

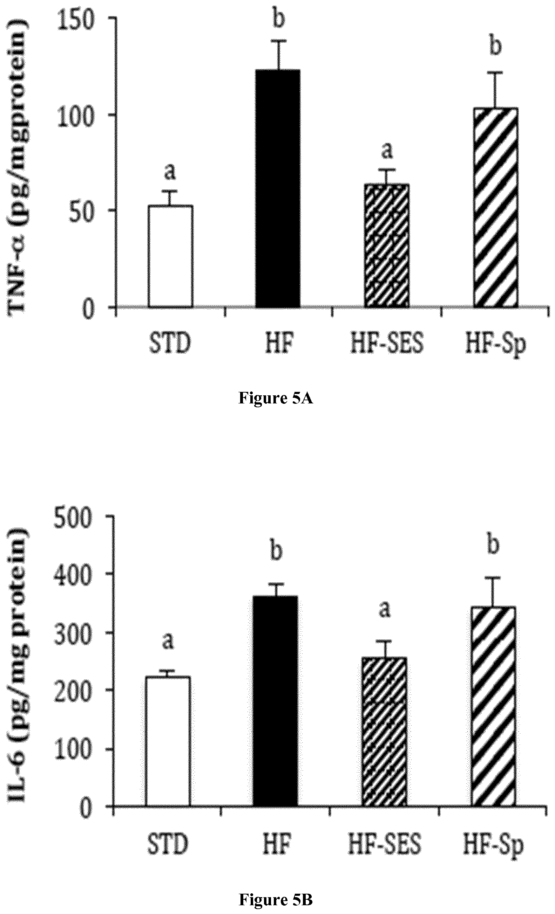

**Figures 4A à 4D** **:** Evaluation histologique de stéatose hépatique de hamsters nourris, respectivement, avec un régime alimentaire standard (STD, Figure 4A), HF (Figure 4B), HF-SES (Figure 4C) ou un régime HF-Sp (Figure 4D) pendant 12 semaines. Des sections représentatives de tissu hépatique (grossissement x40) sont montrées. S : capillaire sinusoidal, H : hépatocyte, N : noyau, MS : stéatose microvasculaire, PN : cellules polynucléaires. **Figures 5A et 5B** **:** Représentation de l'état inflammatoire indexé sur la concentration en cytokines dans le foie, respectivement le niveau de TNF-alpha (Figure 5A) ou de IL-6 (Figure 5B) de hamsters ayant reçu un régime alimentaire STD, HF, HF-SES ou HF-Sp pendant 12 semaines. Les valeurs indiquent la moyenne +/- SEM (n=6). Pour chacun des régimes diététiques, les barres avec différentes lettres en index sont significativement différentes (p<0,05).

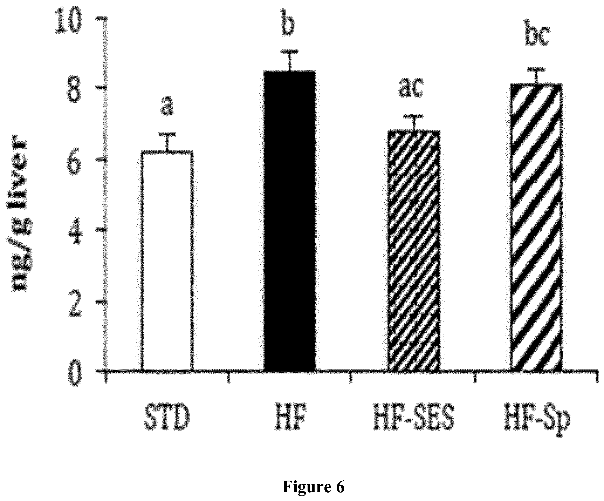

**Figure 6** **:** Activité NFκB (en ng par g de foie) dans le foie de hamsters ayant reçu un régime alimentaire STD, HF, HF-SES ou HF-Sp pendant 12 semaines. Les valeurs indiquent la moyenne +/- SEM (n=6). Pour chacun des régimes diététiques, les barres avec différentes lettres en index sont significativement différentes (p<0,05).

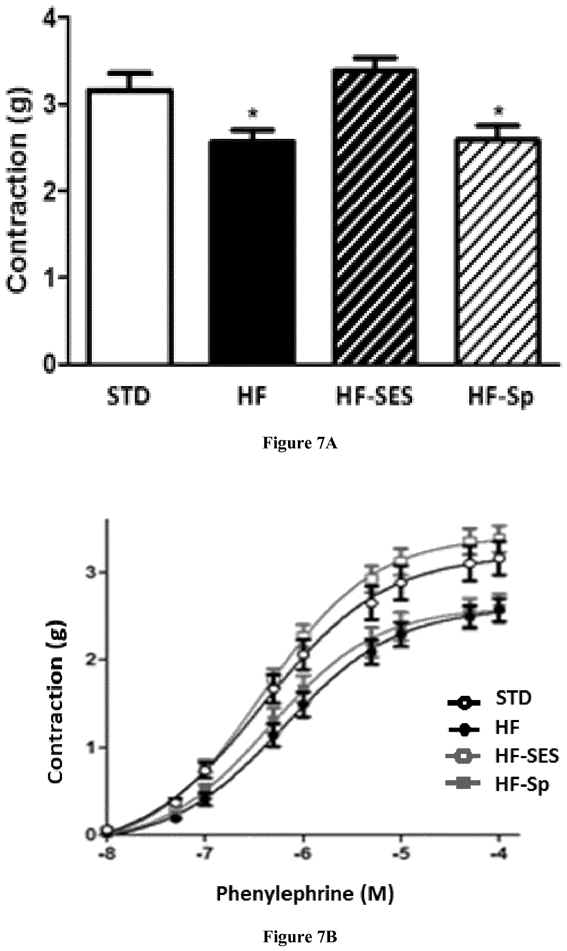

**Figures 7A et 7B** : Représentation de la réponse contractile à la phényléphrine. Histogramme représentant la contraction (en g) pour chacune des conditions testées, avec de G à D : STD, HF, HF-SES, HF-Sp (Fig. 7A). Représentation de la contraction (en g) pour chacune des conditions testées (STD, HF, HF-SES, HF-Sp) en fonction de la concentration en phényléphrine (en M) (Fig. 7B).

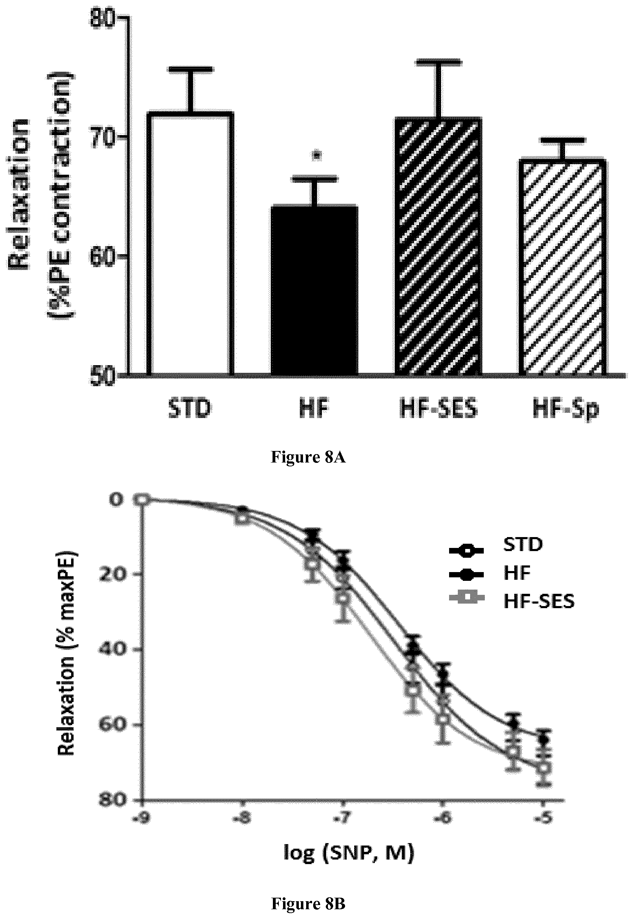

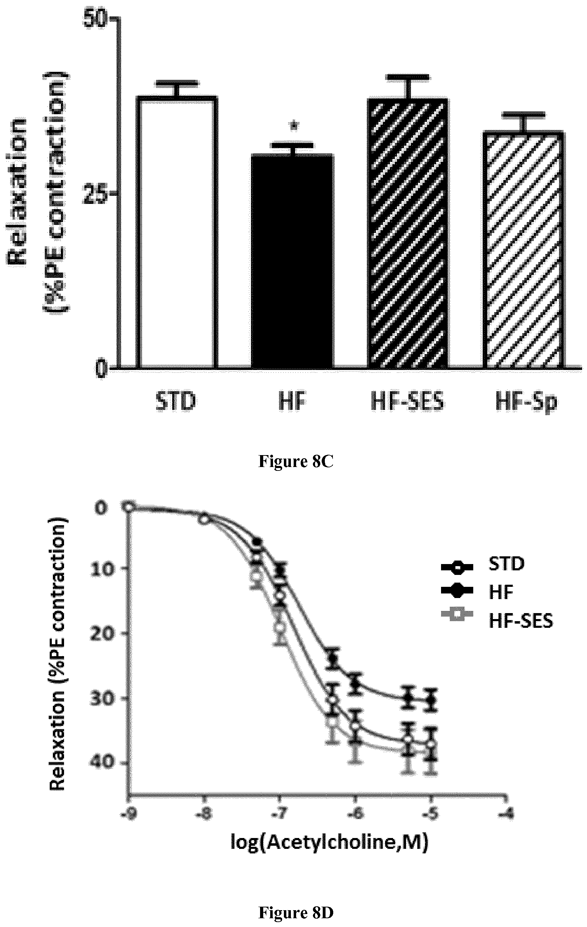

**Figures 8A à 8D** **:** Représentation de la réponse contractile des aortes soumises à des antagonistes de la contraction. Figures 8A et 8B : relaxation en réponse au SNP. Figure 8A : histogramme représentant la relaxation (en % PE de contraction) pour chacune des conditions testées, avec de gauche à droite : STD, HF, HF-SES, HF-Sp. Figure 8B : Représentation de la relaxation (en % max PE) pour chacune des conditions testées (STD (ronds clairs), HF (ronds noirs), HF-SES (carrés clairs) en fonction de la concentration en SNP (en log(SNP), en M). Figures 8C et 8D : relaxation en réponse à l'acétylcholine. Figure 8C : histogramme représentant la relaxation (en % PE de contraction) pour chacune des conditions testées, avec de G à D : STD, HF, HF-SES, HF-Sp. Figure 8D : Représentation de la relaxation (en % max PE) pour chacune des conditions testées (STD (ronds clairs), HF (ronds noirs), HF-SES (carrés clairs) en fonction de la concentration en acétylcholine (en log(acetylcholine), en M).

### EXEMPLES

### Exemple 1 : Caractérisation de l'état métabolique, anti-oxydant et anti-inflammatoire dans un modèle animal

### Production des microalgues

Les spirulines sont obtenues par la mise en culture de spiruline de la souche *Arthrospira platensis* dans un milieu de culture spécifique adapté à partir du milieu Zarouk, caractérisé par un pH compris entre 9 et 10,5, contenant, en g/L : NaHCO₃ 8; K₂SO₄ 1; KNO₃ 2; K₂SO₄ 1; NaCl 5; MgSO₄/7H₂ 0,2; NH₄H₂PO₄ 0,2 ; Ferfol (Fer EDTA) 0,02 et en mg/L : MnCl₂/4H₂O 117; CuSO₄/5H₂O 25; ZnSO₄/4H₂O 250. La culture est réalisée en bassin de 25 m³ sous serre. L'agitation est assurée par des pompes de circulation ou des roues à aubes. L'enrichissement en silicium est réalisé en versant dans les bassins une solution concentrée de métasilicate de sodium (Na₂O₃Si) préalablement dissous en présence de polyphénols dans de l'eau déionisée dans volume de 1 M³, ceci afin de prévenir la polymérisation du métasilicate en silice insoluble. Pour un bassin de 25 M³, 50 kg de métasilicate de sodium sont dissous dans un volume de l'ordre de 1 M³ d'eau dé-ionisée. Après dissolution complète du métasilicate de sodium, 300 g de polyphénols de thé sont ajoutés à la solution et celle-ci est agitée pendant 30 minutes. Les polyphénols de thé sont ajoutés sous la forme d'un extrait de feuilles de thé vert (Camellia sinensis (L.) Kuntze), sous la forme d'une poudre brune dispersible dans l'eau, contenant 98% de polyphénols, 75% de catéchines, 40% de EGCG (Epigallocatechine gallate) et 0,5% de caféine (Naturex, France, Ref. EA140374). Le métasilicate va réagir avec les polyphénols et se stabiliser. La solution est ensuite doucement versée dans le bassin de 25 M³ contenant la culture de spiruline déjà présente à une concentration en biomasse sèche de 0,5g/litre environ. La concentration de métasilicate de sodium dans la solution initiale est de 50 g/litre afin d'obtenir une concentration finale en métasilicate de sodium de 2g/litre de culture dans les 25 M³ du bassin. La concentration de polyphénols est de 0,3 g/litre dans la solution initiale, elle atteint 0,012g/litre dans le bassin de culture.

Après culture de la spiruline en présence de silicium et de polyphénols pendant une durée comprise entre 12 heures et 3 jours, la culture est récoltée en partie ou en totalité. La récolte d'une partie de la culture de spiruline est effectuée par filtration à travers une membrane de maille 40 microns, pressée, extrudée en filaments de 1mm de diamètre puis séchée à 40°C.

La quantité totale en silicium des spirulines ainsi produites est de 1 % de la masse sèche. Cette proportion en silicium est déterminée par dosage gravimétrique. La proportion de silicium présent sous une forme soluble dans l'eau est déterminée par extraction séquentielle en présence de solutions aqueuse d'agents de plus en plus actifs. Le protocole est répété pour toute la série d'agents d'extraction et le culot final résiduel est récupéré pour réaliser un dosage du silicium résiduel insoluble. Si le silicium est présent sous forme métabolisée disponible, il sera tout ou partie solubilisé au cours des extractions successives. La proportion de silicium soluble dans l'eau est comprise entre 30 et 60% du silicium total.

### Expérimentation animale

Vingt-quatre hamsters dorés syriens mâles (Janvier-Labs, Le Genest, Saint-Isle, France) pesant 90 g en moyenne ont été répartis au hasard en quatre groupes de six animaux. Ils ont été placés à 23 ± 1°C, soumis à une photopériode de 12L/12D et traités dans le respect des règles de l'Union européenne et conformément aux lignes directrices du NIH (National Research Council, 1985) ainsi que du Comité de protection des animaux à l'Université de Montpellier (France). Un groupe a reçu un régime standard (STD), les trois autres groupes ont reçu pendant 12 semaines un régime hyperlipidique (HF), comportant 200 g/kg de caséine, 3 g/kg de L- méthionine, 393 g/kg d'amidon de maïs, 154 g/kg de saccharose, 50 g/kg de cellulose, 100 g/kg d'huile de coco hydrogénée, 2 g/kg de cholestérol, 35 g/kg de mélange minéral et 10 mg/kg de mélange vitaminique. Les hamsters de chaque groupe ont reçu par gavage soit de l'eau (régime STD, et régime HF), soit de la spiruline native non enrichie (régime HF, groupe HF -SP), soit de la spiruline enrichie en silicium (régime HF, groupe HF- SES). Dans tous les cas, le gavage intragastrique (1ml/jour/hamster) a été réalisé à partir de spiruline (SP ou SES) en suspension dans l'eau et correspondait à une dose de spiruline de 57 mg/kg de poids corporel.

### Procédures analytiques

A la fin de la période expérimentale (12 semaines), les hamsters restent à jeun pendant une nuit. Des échantillons de sang sont prélevés par ponction cardiaque et le plasma est préparé par centrifugation à 2000 g pendant 10 min. Le cholestérol total (CT) et le cholestérol HDL (HDL - C) ont été déterminés en utilisant des kits enzymatiques (CH 200 et CH 203 respectivement, Randox Laboratories Ltd, Crumlin, Royaume-Uni). Le cholestérol des VLDL et LDL a été précipité en utilisant le réactif au phosphotungstate (Weingand & Daggy, 1990), puis le cholestérol-HDL a été mesuré dans le surnageant. Les triglycérides plasmatiques (TG) ont été mesurés en utilisant un kit Randox (TR 1697) et la glycémie au moyen d'une technique enzymatique (KonePro, Konelab, Evry -Les- Lys, France) utilisant des réactifs fournis par Thermo Electron Corporation (Cergy Pontoise, France). L'activité de la paraoxonase (PON) a été déterminée avec le paraoxon comme substrat et mesurée par l'augmentation de l'absorbance à 412 nm due à la formation de 4-nitrophénol, selon la technique selon la technique de Jaouad et al. (2006). L'insulinémie a été déterminée à l'aide d'un kit ELISA (Mercodia AB, Uppsala, Suède). La concentration en « monocyte chemo-attractant protein-1 » (MCP- 1) plasmatique a été mesurée en utilisant un kit Elisa spécifique selon les instructions du fabricant (R & D Systems Europe, Lille, France). L'évaluation de la résistance à l'insuline (HOMA -IR) a été déterminée à partir des valeurs de l'insulinémie et de la glycémie à jeun selon Matthews et al. (1985) : HOMA -IR = ((glygémie à jeun (mmol/L) x insulinémie à jeun (mU/L)) / 22,5.

Le foie a été excisé, pesé et sectionné. Certains échantillons ont été utilisés pour l'histologie, tandis que d'autres ont été stockés à -80 °C jusqu'à utilisation ultérieure. Pour l'analyse anatomo-pathologique, des échantillons de foie ont été fixés dans du formaldéhyde à 10% et inclus dans de la paraffine. Des coupes de 3 micromètres d'épaisseur ont été préparées puis déparaffinées et colorées à l'hématoxyline et à l'éosine. Des échantillons de foie non fixés ont été homogénéisés dans du tampon phosphate de potassium 0,1M refroidi à la glace (pH 7,4) et l'homogénat a été centrifugé à 13000 g pendant 15 min à 4 ° C. Le surnageant a ensuite été stocké à -80 ° C pour mesure ultérieure d'une part des activités superoxyde dismutase (SOD) et glutathion peroxydase (GPx) sur un automate Pentra 400 (Horiba ABX , Montpellier , France) en utilisant les kits Ransod n° SD125 et Ransel n° RS505 respectivement (Randox Laboratories Ltd, Crumlin, Royaume-Uni) et d'autre part pour quantifier les taux d' IL- 6 et de TNF-α en utilisant des kits ELISA spécifiques selon les instructions du fabricant (R & D Systems Europe, Lille, France). Pour mesurer l'activité du facteur nucléaire NFκB, le tissu hépatique a été homogénéisé dans un tampon hypotonique (pH 7,9) contenant 20 mM de HEPES, 10 mM EDTA, 10 mM de KCl, un cocktail d'inhibiteurs de protéase à 1%, 0,1% de dithiothréitol et 0,1% Igepal. Des extraits nucléaires ont été ensuite obtenus par homogénéisation du culot dans un tampon de lyse (pH 7,9) contenant 20 mM HEPES, 1 mM EDTA, 200 mM NaCl, 10% glycerol, 1 mM dithiothréitol et un cocktail d'inhibiteurs de protéase à 1%. L'activité de NFκB a été déterminée à partir d'extraits nucléaires en utilisant un dosage immunologique commercial (Active Motifs, Rixensart, Belgique).

La production hépatique et cardiaque de l'anion superoxyde (O₂ °⁻) a été évaluée par l'intensité de la chimiluminescence de lucigénine (10 microM lucigénine), mesurée avec un luminomètre (Perkin Elmer Wallac, Victor, Turku, Finlande) comme décrit précédemment par Sutra *et al.* (2007). Les résultats ont été exprimés en unités relatives de luminescence (RLU) par mg de protéine.

### Résultats expérimentaux

### Absorption de nourriture et gain de poids

Le tableau 1 présente les résultats obtenus : Poids corporel, absorption de nourriture, cholesterol plasmatique, triglycérides, activité paraoxonase (PON) et modèle homéostatique de résistance à l'insuline (HOMA-IR) chez des hamsters recevant un régime standard avec de l'eau (STD), un régime riche en grasses et de l'eau (HF), un régime riche en graisse et de la spiruline (HF-Sp) ou un régime riche en graisses et de la spiruline enrichie en silicium soluble (HF-SES) pendant 12 semaines.

Les hamsters nourris avec une alimentation HF (groupe High Fat contrôle) ont augmenté leur consommation alimentaire de manière significative de 27% (p = 0,0062) et leur gain de poids corporel de ∼ 80% (p <0,0007) par rapport aux hamsters nourris au régime standard (STD) (Tableau 1). Les groupes recevant une alimentation HF et de la spiruline, enrichie ou non avec le silicium, à savoir les groupes HF-SES et HF-Sp, ont un gain de poids nettement inférieur à celui du groupe de contrôle de HF, soit 52% (p = 0,0001) et 44% (p = 0,0008) respectivement, bien qu'il n'y ait pas de différence de prise alimentaire entre les trois groupes (Tableau 1). Les hamsters des groupes HF-SES et HF-Sp ont une plus faible consommation de nourriture que les hamsters HF. La consommation de spiruline ou de spiruline enrichie en silicium n'entraîne aucun problème de santé chez les animaux.

**Tableau 1**

| | STD | HF | HF-SES | HF-Sp |
|---|---|---|---|---|
| Gain de poids corporel (g) | 32.7±5.2^{a} | 58.7±7.2^{b} | 28.6±4.3^{a} | 33.0±2.8^{a} |
| Absorption de nourriture (g/jour) | 4.9±0.2^{a} | 6.2±0.6 ^{b} | 5.5±0.6 ^{b} | 5.5±0.5 ^{b} |

| Taux plasmatique | | | | |
|---|---|---|---|---|
| TC (mmol/L) | 3.19±0.15^{a} | 7.26±0.26^{b} | 6.11±0.37^{c} | 6.81±0.23^{b} |
| HDL-C (mmol/L) | 1.97±0.23^{a} | 3.26±0.18^{b} | 2.52±0.16^{c} | 3.03±0.12^{b} |
| LDL-C (mmol/L) | 1.22±0.23^{a} | 4.00±0.32^{b} | 3.59±0.37^{b} | 3.78±0.24^{b} |
| TG (mmol/L) | 1.06±0.06^{a} | 1.73±0.27^{b} | 1.14±0.03^{a} | 1.60±0.29^{ab} |
| PON (U/mL) | 172.8±10.2^{a} | 118.2±11.6^{b} | 146.9±9.9^{ab} | 151.0±7.7^{a} |
| Glucose (mmol/L) | 7.78±0.13^{a} | 10.88±0.67^{b} | 12.28±0.91^{b} | 12.04±1.46^{b} |
| Insuline (pmol/L) | 155.4±43.1^{a} | 456.7±79.8^{b} | 127.8±43.9^{a} | 178.0±30.5^{a} |
| HOMA-IR | 9.98±2.90^{a} | 22.55±4.90^{b} | 9.92±0.80^{a} | 12.06±2.53^{c} |

Les valeurs sont des moyennes ± SEM (n = 6). Pour chaque régime alimentaire, les moyennes associées à des lettres différentes diffèrent, p < 0.05. TC : cholestérol plasmatique total, HDL-C : HDL-cholesterol, LDL-C : LDL- cholesterol, TG : triglycérides, PON : activité Paraoxonase et HOMA-IR (résistance à l'insuline) ont été mesurées après un jeûne pendant une nuit, à la fin d'une période de traitement de 12 semaines.

### Analyse des lipides plasmatique

Les hamsters nourris au régime HF ont montré une dyslipidémie significative par rapport à ceux du groupe standard (STD) (Tableau 1). L'alimentation HF-Sp n'a pas induit une amélioration de ces paramètres alors que l'alimentation HF-SES a amélioré ces paramètres, excepté pour le HDL-C. On observe une diminution significative du cholestérol total et des triglycérides chez les hamsters recevant un régime HF-SES.

Par rapport au groupe STD, on observe également dans le groupe HF un taux de glucose plasmatique à jeun de 40% plus élevé (p = 0,0256) et une augmentation de 194% de la concentration d'insuline (p <0,0002), conduisant à une augmentation de 126% de la résistance à l'insuline, tel que mesuré par HOMA-IR (p = 0,0075) (Tableau 1). Bien que l'alimentation HF-SES ou HF-Sp n'ait pas amélioré la glycémie, HF-SES a particulièrement induit une diminution significative de l'insuline plasmatique (72%, p <0,0001) et HOMA-IR (56%, p = 0,0010) jusqu'à des niveaux comparables à ceux observés dans le groupe STD.
L'activité PON plasmatique est résumée dans le tableau 1. Dans le groupe de HF, son activité a été réduite d'environ 31,6% par rapport au groupe STD (p = 0,0010). Dans les groupes HF-SES et HF-SP on observe une amélioration significative de l'activité PON, d'environ 26% en moyenne par rapport aux témoins.

Dans le groupe HF, le taux plasmatique de MCP-1 est significativement élevée de 420% (p <0,0001) par rapport au groupe STD (Figure 1). Cette concentration a été significativement réduite de 28% dans le groupe HF-SES (p = 0,0380), tandis qu'un effet plus faible est observé avec HF-Sp

### Stress oxydant cardiaque et hépatique

Le stress oxydant se définit comme le déséquilibre entre les oxydants et les défenses antioxydantes.

Dans le foie, la production d'anion superoxyde- (activité NADPH oxydase) a augmenté de 98% dans le groupe HF comparé au groupe standard (p<0,0001). Cette production a été significativement diminuée chez les hamsters ayant reçu un régime HF-SES et HF-Sp, avec une diminution respective de 64% et 42%, par rapport aux hamsters HF (Figure 2A). Dans le ventricule cardiaque gauche, la production d'anion superoxyde a été augmentée significativement de 101% (p<0,0001) dans le groupe HF comparé au groupe standard. Cette production a été significativement diminuée de 35% (p=0,006) et 20% (p<0,0001) chez les hamsters recevant HF-SES et HF-Sp respectivement, par comparaison avec le groupe HF (Figure 2B).

### Activité des enzymes anti-oxydantes hépatiques

Dans le groupe contrôle HF, les activités superoxyde dismutase (SOD) et glutathion peroxydase (GPx) ont augmenté de 35% et 75%, respectivement par comparaison avec le groupe standard (p=0,0171 et p<0,0001 respectivement) (Figures 3A et 3B). Cependant, dans les groupes HF-SES et HF-Sp, l'activité des deux enzymes a chuté de 36% (p=0,0017) et 61% (p<0,0001) respectivement pour la SOD, et de 23% (p=0,01861) et 24% (p=0,0049) respectivement pour la GPx, comparé au groupe HF.

### Stéatose et inflammation hépatique

Les hamsters du groupe standard ne montrent aucun signe histologique de stéatose hépatique (Figure 4A). Les hamsters athérogènes nourris sans spiruline (Figure 4B) illustrent le développement d'une stéatose modérée à sévère, avec une inflammation indiquée par la présence répandue de cellules polynucléaires (PN), par comparaison avec des animaux ayant reçu un régime alimentaire standard (Figure 4A). La stéatose hépatique n'est pas affectée chez les animaux nourris avec de la spiruline ou de la spiruline enrichie en silicium (Figures 4C et 4D), bien que les cellules polynucléaires soient diminuées par rapport aux animaux contrôle.

Les concentrations de TNF-α et d'IL-6 dans le foie du groupe HF ont été significativement augmentées de 135% (p <0,0001) et 64% (p = 0,0015), respectivement, par rapport au groupe STD (Figure 5A et B). Cet état inflammatoire de bas grade n'a été inversé que par l'administration de la spiruline enrichie en silicium au groupe HF-SES qui présentait des niveaux de cytokines comparables à ceux du groupe STD.

Dans le groupe HF, l'activité NFκB a augmenté de manière significative (36%, p = 0,0051) par rapport au groupe STD. Seule l'administration de SES (groupe HF-SES) a induit une diminution significative de l'activité NFκB hépatique (21%, p = 0,0259) et a atteint le niveau du groupe STD (Figure 6).

### Conclusions

Les effets de la spiruline enrichie en silicium (SES) sur le développement de l'athérosclérose précoce induite par une haute teneur en graisses (HF) dans l'alimentation de hamsters syriens ont été étudiés. En raison de la nature multifactorielle de l'évolution de l'athérosclérose, les caractéristiques métabolique, oxydante et inflammatoire ont été examinées. Les résultats démontrent les effets bénéfiques de la spiruline enrichie en silicium dans la prévention de désordres lipidiques ainsi que des déséquilibres oxydatifs et inflammatoires induits par l'alimentation HF.

Comme prévu, le régime alimentaire HF, caractérisé par un taux élevé d'acides gras saturés et de cholestérol a induit une augmentation significative des taux de cholestérol plasmatique après 13 semaines. L'administration de SES a partiellement empêché cette augmentation. Dans une étude précédente (Riss *et al.,* 2007), il a été montré que l'administration de la spiruline enrichie en sélénium à des hamsters hypercholestérolémiques n'a pas induit une amélioration de la dyslipidémie après 13 semaines.

Le régime alimentaire HF a également induit une résistance à l'insuline (HOMA-IR), caractérisée par un taux de glucose et d'insuline plasmatique élevé. Cependant, l'administration de SES diminue l'insulinémie, résultant en une diminution de la résistance à l'insuline plus prononcée qu'après l'administration de spiruline non enrichie en silicium. Le régime HF induit une diminution de la sensibilité à l'insuline qui est généralement associée à d'autres troubles tels que le stress oxydatif et l'inflammation, les différents facteurs contribuant au développement de l'athérosclérose.

Ici, le régime HF a effectivement entraîné un stress oxydatif cardiaque et hépatique, comme en témoigne la surproduction de l'anion superoxyde (O₂ °⁻). La NADPH oxydase est une source majeure de production d'anion superoxyde (O₂ °⁻) et toute augmentation de son activité conduit ici à un stress oxydatif. Ceci est en accord avec des études précédentes où l'alimentation HF induit une augmentation de l'activité et de l'expression de la sous-unité p22^{phox} de la NADPH oxydase. Ici, la spiruline seule et SES améliorent l'état oxydatif dans le foie et le coeur, diminuant la production d'anion superoxyde. Dans chaque organe, l'addition de SES présentait le meilleur effet.

Dans le groupe HF, les activités SOD hépatique GPx ont été efficacement améliorées par l'administration de spiruline enrichie en silicium (SES), par rapport au groupe standard. Toutefois, cette induction n'a pas empêché la surproduction hépatique d'anion superoxyde, ce qui suggère que la production de ROS pourrait submerger les mécanismes de défense enzymatiques, ce déséquilibre résultant dans un stress oxydatif du foie. Les hamsters nourris soit avec la spiruline seule ou avec SES ont montré une baisse des activités GPx et SOD similaire à celles du groupe STD. L'administration de la spiruline seule et de SES a réduit la dépendance sur les antioxydants endogènes.

L'activité PON est un autre marqueur de stress oxydatif. PON est synthétisé dans le foie et sécrété dans le plasma sous forme de protéine associée au HDL et joue un rôle majeur dans l'activité antioxydante des HDL. Il est capable d'hydrolyser les phospholipides oxydés et les produits de peroxydation des lipides, ce qui inhibe l'oxydation des LDL et HDL. En conséquence, PON pourrait protéger contre l'athérosclérose, et la modulation de son activité fait partie des cibles thérapeutiques actuelles. Le stress oxydatif associé aux régimes HF est connu pour diminuer l'activité PON, ici cette activité est considérablement réduite dans le groupe HF. La fourniture de spiruline a partiellement permis de prévenir une telle chute de l'activité PON, ce qui renforce leur potentiel antioxydant.

Globalement, SES a présenté des propriétés antioxydantes comme en témoigne la diminution de l'activité de la NADPH oxydase avec leur effet d'épargne sur les enzymes antioxydantes. Il est bien connu que la co-existence d'un état d'insulinorésistance et de stress oxydatif hépatique sont liés au développement de la stéatose hépatique non alcoolique. Ici, le groupe HF a effectivement développé une stéatose hépatique, comme en témoigne l'accumulation de lipides représentés par l'analyse histologique. Malgré la présence de cellules polynucléaires dans le foie, aucun signe de fibrose n'a été identifié, suggérant que l'étape de la stéatohépatite non alcoolique (NASH) n'avait pas encore été atteinte. L'état d'inflammation de bas grade dans le foie d'animaux HF est confirmé par la surproduction de cytokines (TNF-α et IL-6), en moyenne de 90% supérieure à celle du groupe STD. Bien que les groupes recevant de la spiruline seule ou de SES n'ont montré aucune amélioration de la stéatose, ils présentent moins de cellules polynucléaires que le groupe HF. En outre, la surproduction de TNF-α et IL-6 ont été prévenus et ont atteint le niveau de référence (STD) dans le groupe SES seulement.

NFκB, un facteur de transcription sensible au stress oxydatif, est un régulateur clé de l'expression de nombreux gènes impliqués dans la réponse immunitaire et inflammatoire et les cytokines induites par NFκB contribuent largement aux maladies inflammatoires, y compris l'athérosclérose. L'amélioration de l'état inflammatoire dans le groupe SES, comme en témoigne la réduction de l'IL-6 et TNF-α, pourrait passer par une inhibition de l'activité de NFκB. Dans notre étude, seule la supplémentation SES a significativement prévenu l'activation des NFκB au niveau du foie et la production inhérente de cytokines pro-inflammatoires. L'effet antioxydant de SES a probablement contribué à l'inhibition de l'activation de NFκB. En outre MCP-1 joue un rôle critique dans le développement de maladies cardio-vasculaires. MCP-1, par son activité chimiotactique, induit une diapédèse des monocytes à partir de la lumière vers l'espace sous-endothélial où ils deviennent des cellules spumeuses, initiant la formation de stries grasses qui mènent à la formation de la plaque athérosclérotique. Ici, la supplémentation en SES réduit le niveau de MCP-1 dans le plasma, ce qui suggère que SES pourrait limiter le développement de l'athérosclérose.

En conclusion, il a été démontré que la spiruline enrichie en silicium (SES) a été en mesure d'inhiber les troubles athérogènes liées à l'alimentation. SES a amélioré le niveau de résistance à l'insuline ainsi que l'état oxydatif et inflammatoire du foie, qui sont étroitement liées au développement de l'athérosclérose. La sensibilité à l'insuline a un lien de cause à effet avec la réduction du stress oxydatif, et inversement. De même, les propriétés antioxydantes de SES ont contribué à améliorer l'état inflammatoire du foie. Les cibles moléculaires impliquées dans ces effets bénéfiques incluent l'inhibition de NFκB. Dans l'ensemble, l'administration de SES montre des effets préventifs sur les troubles liés à l'athérogénèse, notamment à l'hypercholestérolémie, l'hypertriglycéridémie, le stress oxydatif et l'inflammation de bas grade, soulignant la valeur ajoutée de silicium par rapport à la spiruline non enrichie en silicium (spiruline seule). Un apport adéquat de cette spiruline de silicium enrichi pourrait être utile dans la prévention des maladies cardio-vasculaires.

### Exemple 2 : Réactivité vasculaire d'aortes isolées de hamsters soumis à un régime riche en graisses, avec supplémentation journalière en spiruline, seule ou enrichie en silicium Conditions expérimentales

Comme dans l'exemple 1, les animaux ont reçu un régime standard équilibré (STD), un régime riche en graisses hydrogénées et en cholestérol (HF), un régime riche en graisses hydrogénées et en cholestérol et une dose journalière de spiruline enrichie en silicium à 1% (soit 0,57 mg de silicium par kg de poids d'animal) (HF-SES) ou un régime riche en graisses hydrogénées et en cholestérol et une dose journalière de spiruline non enrichie équivalente à la dose de spiruline du régime HF-SES (HF-Sp). Par « mg de silicium par kg de poids d'animal, il est entendu la quantité totale de silicium reçue par l'animal, cette quantité ayant été doées par gravimétrie.

Les expériences de réactivité vasculaire ont été réalisées sur les aortes par la méthode de mesure des variations de tension isométriques à l'aide d'un myographe (Système à organe isolé, EMKA Technologie, France). Après prélèvement, le tissu immergé dans un milieu physiologique est disséqué et nettoyé des tissus adipeux et conjonctif. L'aorte est ensuite coupée en cinq segments ou anneaux de 2 à 3 mm de longueur. Sur certains segments, l'endothélium est détruit par frottement consécutif à l'introduction d'un cure-dent dans la lumière de l'artère. Les anneaux sont montés entre deux crochets métalliques reliés à un capteur de force, immergés dans une cuve du myographe et maintenus à 37°C sous oxygénation permanente. Chaque segment est soumis à une période d'équilibre de 60 minutes à la tension initiale optimale de 1g. Les variations de tension sont enregistrées en réponse à différents agonistes et antagonistes. La viabilité de chaque anneau est tout d'abord évaluée par l'augmentation de la tension induite après addition d'une dose sub-maximale de phényléphrine (PE- 10 µml/L), la présence d'endothélium étant ensuite validée par la relaxation consécutive à l'addition d'acétylcholine (Ach- 1 µmol/L). Après plusieurs lavages et une nouvelle période de stabilisation de 30 minutes, les doses réponses sont réalisées pour évaluer dans un premier temps la fonction contractile en réponse à des doses cumulées de phényléphrine (10 nmol/L à 100 µmol/L). La fonction endothéliale est ensuite déterminée en étudiant la relaxation induite par l'addition de doses cumulées d'acétylcholine (10 nmol/L à 10 µmol/L). La relaxation endothélium indépendante induite par le sodium nitroprusside (SNP) est étudiée par l'addition de doses cumulées de SNP (1 nmol/L à 100 µmol/L) sur des anneaux dé-enthotélialisés et préalablement contractés avec la PE (10 µmol/L). Chaque protocole a été réalisé en triplicata sur des tissus provenant de 6 animaux différents.

### Résultats expérimentaux

Comme indiqué dans les figures 7A et 7B, on observe une diminution de la contraction à la phényléphrine avec le régime HF en comparaison du régime standard (STD), cette diminution est prévenue avec la supplémentation en spirulines enrichies (HF-SES) et non avec la supplémentation en spirulines seules (HF-Sp). Cette diminution traduit une altération de la fonction contractile, cette altération est prévenue avec le régime SES.

L'étude de la réponse contractile des aortes soumises à des antagoniste de la contraction (=relaxation) tels que le SNP (nitroprussiate de sodium, mimétique du monoxyde d'azote (NO)) et l'acétylcholine montre qu'on observe une moins bonne relaxation induite par le SNP avec le régime HF (Figures 8A et 8B). Cette diminution de l'effet du SNP traduit une altération de la fonction contractile des artères. Cette altération est prévenue avec le régime SES puisque la relaxation induite par le SNP est identique dans le groupe HF-SES et le groupe STD.

On observe également une moins bonne relaxation à l'acétylcholine avec le régime HF (Figures 8C et 8D). Cet effet sur la relaxation traduit une dysfonction endothéliale, cette dysfonction est prévenue avec le régime SES. La réponse à l'acétylcholine n'est pas modifiée pour les animaux du groupe HF-SES par rapport aux animaux STD.

### Conclusion

Des altérations de la fonction endothéliale et de la vasomotricité des artères sont observées chez l'animal alimenté avec le régime HF. Ces altérations sont prévenues par une supplémentation en spirulines enrichies en silicium mais pas par une supplémentation en spirulines non enrichies en silicium.

### REFERENCES BIBLIOGRAPHIQUES

Loeper, J., et al., 1979, Atherosclerosis, 33, 357-408.
Maehira, F., et al., (2011). Nutr. Res., 31, 147-56.
Refitt, D. M., et al., 2003, Bone, 32, 127-135.
Riss, J., et al. (2007). J. Agric. Food Chem, 55, 7962-7967.
Schwarz, K. (1977). Lancet, 1, 454-456.
Seaborne, C. D., & Nielsen, F. H. (1993). Nutr. Today, 28, 13-18. 480
Sutra, T *et al*., (2007) May 16;55(10):4258-63.
Vinson et al., (2001), Atherosclerosis, 156, 67-72
Wang et al., (2001), Eur. J. Pharmacol., 427, 285-293.
Organisation Mondiale de la Santé / World Health Organization « Global health risks: mortality and burden of disease attributable to selected major risks », 2004, WHO Library Cataloguing-in Publication-data.

## Revendications

1. Composition comprenant une microalgue enrichie en silicium sous une forme soluble dans l'eau, destinée à être utilisée pour la prévention ou le traitement d'une pathologie liée au syndrome métabolique, choisie parmi le diabète de type 2, l'obésité nutritionnelle, les dyslipidémies, les pathologies vasculaires et cardiovasculaires, et l'inflammation.

2. Composition comprenant une microalgue enrichie en silicium sous une forme soluble dans l'eau, destinée à être utilisée selon la revendication 1, dans laquelle ladite microalgue est la spiruline.

3. Composition comprenant une microalgue enrichie en silicium sous une forme soluble dans l'eau, destinée à être utilisée selon l'une des revendications 1 ou 2, **caractérisée en ce qu'**elle est formulée pour être administrée par voie orale.

4. Composition comprenant une microalgue enrichie en silicium sous une forme soluble dans l'eau, destinée à être utilisée selon la revendication 3, **caractérisée en ce qu'**elle se présente sous une forme choisie parmi : gélule, capsule, comprimé, granule, tablette, poudre ou solution buvable.

5. Composition comprenant une microalgue enrichie en silicium sous une forme soluble dans l'eau, destinée à être utilisée selon l'une des revendications 1 à 4, **caractérisée en ce qu'**elle est formulée seule ou avec au moins un excipient ou additif, pour être administrée comme médicament, comme nutraceutique ou comme complément alimentaire.

6. Composition comprenant une microalgue enrichie en silicium sous une forme soluble dans l'eau, destinée à être utilisée selon la revendication précédente, **caractérisée en ce qu'**elle est formulée avec au moins un adjuvant pharmaceutiquement acceptable choisi parmi les épaississants, les conservateurs, les parfums, les colorants, filtres chimiques ou minéraux, agents hydratants, eaux thermales.

7. Composition comprenant une microalgue enrichie en silicium sous une forme soluble dans l'eau, destinée à être utilisée selon l'une des revendications précédentes, pour son utilisation pour la prévention ou la diminution de la surcharge pondérale, pour la prévention ou la diminution de l'adipogenèse, ou en tant qu'agent antioxydant.

8. Composition comprenant une microalgue enrichie en silicium sous une forme soluble dans l'eau, destinée à être utilisée selon l'une des revendications précédentes, **caractérisée en ce que** la proportion de silicium total dans ladite composition est comprise entre 0,05% et 10%, de la masse sèche de microalgue présente dans ladite composition.

9. Composition comprenant une microalgue enrichie en silicium sous une forme soluble dans l'eau, destinée à être utilisée selon l'une des revendications précédentes, **caractérisée en ce que** la dose de silicium administrée est comprise entre 0,3 et 1 mg/kg/jour.

## Patentansprüche

1. Zusammensetzung, die eine Mikroalge umfasst, welche an Silicium in einer wasserlöslichen Form angereichert ist, wobei sie dazu bestimmt ist, zur Verhütung oder Behandlung einer Erkrankung verwendet zu werden, die mit dem metabolischen Syndrom in Verbindung steht, ausgewählt aus Diabetes vom Typ 2, ernährungsbedingter Fettleibigkeit, Fettstoffwechselstörungen, Erkrankungen der Gefäße und der Herzgefäße sowie Entzündungen.

2. Zusammensetzung, die eine Mikroalge umfasst, welche an Silicium in einer wasserlöslichen Form angereichert ist, wobei sie dazu bestimmt ist, gemäß Anspruch 1 verwendet zu werden, wobei es sich bei der Mikroalge um Spirulin handelt.

3. Zusammensetzung, die eine Mikroalge umfasst, welche an Silicium in einer wasserlöslichen Form angereichert ist, wobei sie dazu bestimmt ist, gemäß einem der Ansprüche 1 oder 2 verwendet zu werden, **dadurch gekennzeichnet, dass** sie für eine Verabreichung auf oralem Wege formuliert ist.

4. Zusammensetzung, die eine Mikroalge umfasst, welche an Silicium in einer wasserlöslichen Form angereichert ist, wobei sie dazu bestimmt ist, gemäß Anspruch 3 verwendet zu werden, **dadurch gekennzeichnet, dass** sie in einer Form vorliegt, die aus den folgenden ausgewählt ist: Gelatinekapsel, Kapsel, Tablette, Granulat, Buccaltablette, Pulver oder trinkfertige Lösung.

5. Zusammensetzung, die eine Mikroalge umfasst, welche an Silicium in einer wasserlöslichen Form angereichert ist, wobei sie dazu bestimmt ist, gemäß einem der Ansprüche 1 bis 4 verwendet zu werden, **dadurch gekennzeichnet, dass** sie, allein oder mit mindestens einem pharmazeutischen Hilfsstoff oder Zusatzstoff, für eine Verabreichung als Arzneimittel, als Nutrazeutikum oder als Nahrungsergänzungsmittel formuliert ist.

6. Zusammensetzung, die eine Mikroalge umfasst, welche an Silicium in einer wasserlöslichen Form angereichert ist, wobei sie dazu bestimmt ist, gemäß dem vorhergehenden Anspruch verwendet zu werden, **dadurch gekennzeichnet, dass** sie mit mindestens einem pharmazeutisch unbedenklichen Adjuvans formuliert ist, welches aus den Verdickungsmitteln, den Konservierungsstoffen, den Duftstoffen, den Farbstoffen, den chemischen oder mineralischen Filtern, den feuchtigkeitsspendenden Mittel, den Thermalwässern ausgewählt ist.

7. Zusammensetzung, die eine Mikroalge umfasst, welche an Silicium in einer wasserlöslichen Form angereichert ist, wobei sie dazu bestimmt ist, gemäß einem der vorhergehenden Ansprüche verwendet zu werden, wobei sie dazu bestimmt ist, zur Verhütung oder Verringerung von Übergewicht, zur Verhütung oder Verringerung der Fettgewebebildung oder als Antioxidans verwendet zu werden.

8. Zusammensetzung, die eine Mikroalge umfasst, welche an Silicium in einer wasserlöslichen Form angereichert ist, wobei sie dazu bestimmt ist, gemäß einem der vorhergehenden Ansprüche verwendet zu werden, **dadurch gekennzeichnet, dass** der Gesamtanteil an Silicium in der Zusammensetzung im Bereich von 0,05 % bis 10 % liegt, bezogen auf die Trockenmasse an Mikroalgen, welche in der Zusammensetzung vorliegt.

9. Zusammensetzung, die eine Mikroalge umfasst, welche an Silicium in einer wasserlöslichen Form angereichert ist, wobei sie dazu bestimmt ist, gemäß einem der vorhergehenden Ansprüche verwendet zu werden, **dadurch gekennzeichnet, dass** die verabreichte Dosis an Silicium im Bereich von 0,3 bis 1 mg/kg/Tag liegt.

## Claims

1. A composition including silicon-rich water-soluble microalgae, for preventing or treating a condition which is linked to the metabolic syndrome and which is selected from type 2 diabetes, nutritional obesity, dyslipidaemia, vascular and cardiovascular diseases, and inflammation.

2. The composition including silicon-rich water-soluble microalgae, for use according to claim 1, wherein said microalgae is spirulina.

3. The composition including silicon-rich water-soluble microalgae, for use according to one of claims 1 or 2, **characterized in that** it is formulated for oral administration.

4. The composition including silicon-rich water-soluble microalgae, for use according to claim 3, **characterized in that** it is in a form selected from: gelatin capsule, capsule, tablet, granule, thin-film strip, powder or drinkable solution.

5. The composition including silicon-rich water-soluble microalgae, for use according to one of claims 1 to 4, **characterized in that** it is formulated, alone or with at least one excipient or additive, to be administered as a drug, nutraceutical or food supplement.

6. The composition including silicon-rich water-soluble microalgae, for use according to the previous claim, **characterized in that** it is formulated with at least one pharmaceutically acceptable adjuvant chosen from thickeners, preservatives, perfumes, dyes, chemical or mineral filters, moisturizing agents, thermal waters.

7. The composition including silicon-rich water-soluble microalgae, for use according to one of the above claims, in order to be used in preventing or reducing overweight, preventing or reducing adipogenesis, or as an antioxidant agent.

8. The composition including silicon-rich water-soluble microalgae, for use according to one of the above claims, **characterized in that** the proportion of total silicon in said composition is between 0.05% and 10% of the dry mass of microalgae present in said composition.

9. The composition including silicon-rich water-soluble microalgae, for use according to one of the above claims, **characterized in that** the administered silicon dose is between 0.3 and 1 mg/kg/day.
